# EUROPÄISCHE PATENTSCHRIFT

(11) **EP 0 655 437 B1**
(45) Veröffentlichungstag und Bekanntmachung des Hinweises auf die Patenterteilung: **30.10.1996**
(21) Anmeldenummer: 94810652.1
(22) Anmeldetag: 16.11.1994
(51) Int. Cl.: C07C 249/12

(54) **Verfahren zur Herstellung O-substituierter Oxime**
Process for the preparation of O-substituted oximes
Procédé pour la préparation d'oximes O-substituées

(30) Priorität: 25.11.1993 CH 3525/93
(43) Veröffentlichungstag der Anmeldung: 31.05.1995
(73) Patentinhaber: CIBA-GEIGY AG, 4002 Basel (CH)
(72) Erfinder: Blaser, Denis, Dr., CH-1870 Monthey (CH)

(56) Entgegenhaltungen:
- US-A- 4 687 849
- ACS SYMPOSIUM SERIES. SYNTHESIS AND CHEMISTRY OF AGROCHEMICALS II, Bd.443, 1991 Seiten 226 - 235 P. KLAUS ET AL. 'Synthesis of the New Graminicide Propaquizafop'

## Beschreibung

Die vorliegende Erfindung betrifft ein Verfahren zur Herstellung von O-ω-Hydroxyalkyloximen durch Umsetzung von Oximen mit Alkylencarbonaten in Gegenwart katalytischer Mengen einer Amidin-oder Pyridinbase.

Diese Oxime stellen wichtige Zwischenverbindungen für herbizid wirksame Stoffe dar, wie sie zum Beispiel in der US-A-4,545,807 und der US-A-4,687,849 beschrieben sind. In der US-A-4,687,849 wird hierbei ein Verfahren zur Herstellung von 2-[(iso-Propylidenamino)oxy]ethanol vorgeschlagen, bei dem Acetonoxim in wässrigem Medium in Gegenwart katalytischer Mengen Ca(OH)₂ mit Ethylenoxid umgesetzt wird. Ein Nachteil dabei ist, dass Ethylenoxid grosstechnisch schwierig und nur mit hohen Sicherheitsvorkehrungen zu handhaben ist, da es hochexplosiv und äusserst giftig ist. Ein weiterer Nachteil ist der Anfall hoher Abwassermengen, die gereinigt werden müssen.
Es ist auch bereits bekannt, dass 2-[(iso-Propylidenamino)oxy]ethanol durch Umsetzung von Acetonoxim mit Ethylencarbonat hergestellt werden kann. Für diese Reaktion können als Katalysatoren Kaliumfluorid und Tetramethylammoniumbromid eingesetzt werden. Dieses Verfahren ist zum Beispiel in R. Klauser et al. in ACS Symposium Ser. 1991, 443, (Synth. Chem. Agrochem. 2), 226-235 beschrieben. Die gleiche Umsetzung ohne Verwendung von Katalysatoren und Lösungsmitteln beschreiben S. I. Hong et al. in J. Polym. Sci. Part A-1, 1972, 10, 3405-19. Selbst bei erhöhten Temperaturen werden nur Ausbeuten von 33% erreicht. Diese Verfahren haben schwerwiegende ökologische und ökonomische Nachteile, z. B. dass ein heterogenes Reaktionsgemisch vorliegt und lange Reaktionszeiten von bis zu 10 Stunden benötigt werden. Durch die Verwendung eines Fluorids wird die grosstechnische Aufarbeitung des Reaktionsgemisches erschwert. Es muss eine Filtration und eine Waschstufe angeschlossen werden. Zusätzlich muss eine Aufarbeitung des Waschwassers durchgeführt werden.

Es wurde nun gefunden, dass man in homogenen organischen Reaktionsmedien bei erheblich verkürzten Reaktionszeiten mindestens gleich hohe Ausbeuten erzielen kann, wenn man organische Amidin-oder Pyridinbasen als Katalysatoren verwendet. Das gewünschte Produkt kann in einfacher Weise mittels Destillation isoliert werden, wobei Lösungsmittel und Katalysator ebenfalls zurückgewonnen und wiederverwendet werden können. Dieses ökologische und ökonomische Verfahren eignet sich daher besonders für den grosstechnischen Maßstab.

Ein Gegenstand der Erfindung ist ein Verfahren zur Hydroxyalkylierung von Aldoximen und Ketoximen durch Umsetzung dieser Oxime mit unsubstituiertem oder mit C₁-C₈-Alkyl substituiertem Ethylen- oder Propylencarbonat in Gegenwart eines Katalysators, das dadurch gekennzeichnet ist, dass man katalytische Mengen einer N-alkylierten, stabilen, organischen Amidinbase oder eines mit sekundärem Amin substituierten Pyridins verwendet.

Stabil bedeutet, dass die Amidinbase oder Pyridinbase bei den gewählten Reaktionsbedingungen, wie zum Beispiel Temperatur und Lösungsmittel praktisch nicht zersetzt wird.

N-alkyliert bedeutet, dass das N-Atom der Aminogruppe der Amidinbase ein-oder zweifach mit C₁-C₈-Alkyl, bevorzugt mit C₁-C₄-Alkyl substituiert ist, oder das N-Atom Teil eines Rings von mono-bis tricyclischem Ringsystem ist.

Bevorzugt wird das Verfahren zur Herstellung von Verbindungen der Formel I 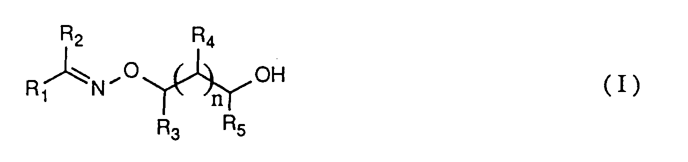 verwendet, worin R₁ und R₂ unabhängig voneinander Wasserstoff, C₁-C₈-Alkyl, C₃-C₈-Cycloalkyl, C₁-C₈-Halogenalkyl, unsubstituiertes oder mit C₁-C₈ Alkyl oder Halogen substituiertes Phenyl, Benzyl, Phenylethyl bedeuten, oder R₁ und R₂ zusammen unsubstituiertes oder mit C₁-C₈-Alkyl oder Halogen substituiertes C₂-C₇-Alkylen bedeuten, R₃, R₄ und R₅ unabhängig voneinander Wasserstoff oder C₁-C₈ Alkyl sind und n eine Zahl 0 oder 1 ist, durch Umsetzung von Verbindungen der Formel II 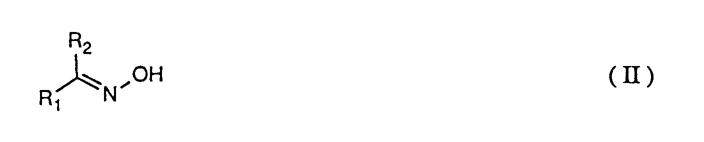
mit Verbindungen der Formel III 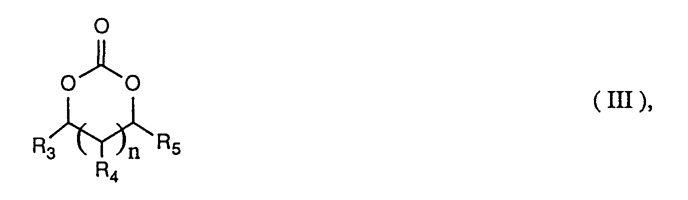 worin R₃, R₄, R₅ und n die vorstehend angegebenen Bedeutungen haben. In den Verbindungen der Formel I steht n bevorzugt für 0.

R₁ und R₂ enthält als Cycloalkyl bevorzugt 5 oder 6 C-Atome. Beispiele für Cycloalkyl sind Cyclopropyl, Dimethylcyclopropyl, Cyclobutyl, Cyclopentyl, Methylcyclopentyl, Cyclohexyl, Cycloheptyl.

Halogen kann im Rahmen der vorliegenden Erfindung für Fluor, Chlor, Brom oder Jod, bevorzugt für Fluor, Chlor oder Brom und besonders bevorzugt für Fluor oder Chlor stehen.

Beispiele für Halogenalkyl, das bevorzugt 1-4 C-Atome enthält, sind: Fluormethyl, Difluormethyl, Trifluormethyl, Chlormethyl, Dichlormethyl, Trichlormethyl, 2,2,2-Trifluorethyl, 2-Fluorethyl, 2-Chlorethyl, 2,2,2-Trichlorethyl, 1,1,2,2-Tetrafluorethyl sowie teilweise oder vollständig chloriertes oder fluoriertes Isopropyl, n-Propyl, n-Butyl, iso-Butyl, sek-Butyl, tert-Butyl, Pentyl-, Hexyl-, Heptyl- und Octyl.

R₃, R₄ und R₅ stellen bevorzugt Wasserstoff oder C₁-C₄-Alkyl dar. Beispiele für Alkyl sind Methyl, Ethyl, n-oder i-Propyl, n-,i-, oder t-Butyl. Besonders bevorzugt bedeuten R₃, R₄, und R₅ Wasserstoff.

In einer bevorzugten Ausführungsform des Verfahrens sind R₁ und R₂ unabhängig voneinander Wasserstoff, C₁-C₈-Alkyl oder R₁ und R₂ bilden zusammen unsubstituiertes C₂-C₇-Alkylen, R₃ und R₅ bedeuten Wasserstoff oder C₁-C₄ Alkyl, wobei n für 0 steht.

Eine besonders bevorzugte Ausführungsform des Verfahrens ist jene, wenn R₁ und R₂ Methyl, Ethyl oder Propyl sind, R₃ und R₄ Wasserstoff bedeuten und n für 0 steht.

Die Amidinbase enthält das Strukturelement -C-N=C-N-C-. Es kann sich um offenkettige Verbindungen, alicyclische Ringe oder bicyclische und tricyclische Ringsysteme handeln, die 4 bis 8, bevorzugt 5 oder 6 Ringglieder enthalten. Die Amidinbase enthält bevorzugt 4 bis 20, besonders bevorzugt 4 bis 14 und ganz besonders bevorzugt 4 bis 10 C-Atome. Das Sekundäramino als Substituent von Pyridin enthält bevorzugt 2 bis 24, besonders bevorzugt 2 bis 18 und ganz besonders bevorzugt 2 bis 12 C-Atome.

Bevorzugt entspricht die organische Amidinbase der Formel IV oder dem mit sekundärem Amin substituierten Pyridin der Formel V 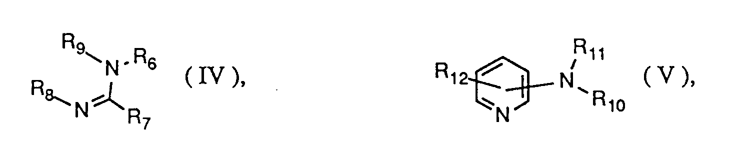 worin R₆, R₇, R₈ und R₉ unabhängig voneinander C₁-C₈-Alkyl oder C₃-C₈-Cycloalkyl bedeuten, oder R₆ und R₇ zusammen einen Kohlenwasserstoffrest mit 3 bis 6 C-Atomen bilden und R₈ und R₉ die vorstehend angegebene Bedeutung haben, oder R₈ und R₉ zusammen einen Kohlenwasserstoffrest mit 2 bis 6 C-Atomen bilden und R₆ und R₇ C₁-C₈-Alkyl oder C₃-C₈-Cycloalkyl bedeuten, oder R₆ und R₇ zusammen einen Kohlenwasserstoffrest mit 3 bis 6 C-Atomen sowie R₈ und R₉ zusammen einen Kohlenwasserstoffrest mit 2 bis 6 C-Atomen bilden,
R₁₀ und R₁₁ unabhängig voneinander C₁-C₁₂-Alkyl sind und R₁₂ für Wasserstoff, C₁-C₈-Alkyl, C₃-C₈-Cycloalkyl oder C₁-C₈-Halogenalkyl steht.

In einer vorteilhaften Ausführungsform des Verfahrens wird als Katalysator eine Verbindung der Formel IV oder V verwendet, wobei R₆ und R₇ zusammen einen Kohlenwasserstoffrest mit 3 bis 6 C-Atomen bilden und R₈ und R₉ C₁-C₈-Alkyl oder C₅-C₆-Cycloalkyl sind, oder R₈ und R₉ zusammen einen Kohlenwasserstoffrest mit 2 bis 6 C-Atomen bilden und R₆ und R₇ C₁-C₈-Alkyl oder C₅-C₆-Cycloalkyl bedeuten, oder R₆ und R₇ zusammen einen Kohlenwasserstoffrest mit 3 bis 6 C-Atomen sowie R₈ und R₉ zusammen einen Kohlenwasserstoffrest mit 2 bis 6 C-Atomen bilden,
R₁₀ und R₁₁ unabhängig voneinander C₁-C₄-Alkyl bedeuten und R₁₂ für Wasserstoff, C₁-C₄-Alkyl, C₅-C₆-Cycloalkyl oder C₁-C₄-Halogenalkyl steht.

Eine besonders vorteilhafte Verfahrensdurchführung besteht darin, dass als Katalysator eine Amidinbase der Formel IV verwendet wird, wobei R₆ und R₇ zusammen einen gesättigten Kohlenwasserstoffrest mit 3 bis 6 C-Atomen und R₈ und R₉ zusammen einen gesättigten Kohlenwasserstoffrest mit 2 bis 6 C-Atomen bilden.

Eine andere bevorzugt verwendete Gruppe von Katalysatoren sind Verbindungen der Formel V wobei R₁₂ Wasserstoff oder C₁-C₈-Alkyl bedeutet, und R₁₀ und R₁₁ gleich sind und für C₁-C₄-Alkyl stehen.

Besonders bevorzugte Katalysatoren für das erfindungsgemässe Verfahren sind Verbindungen der Formel V, wobei R₁₂ Wasserstoff ist und die Gruppe -NR₁₀R₁₁ in 2 oder 4 Stellung gebunden ist, wobei R₁₀ und R₁₁ C₁-C₄-Alkyl sind.

Ganz besonders bevorzugt werden die Verbindungen 1,5-Diazabicyclo[4.3.0]non-5-en, 1,8-Diazabicyclo[5.4.0]undec-7-en und 2-Dimethylaminopyridin sowie 4-Dimethylaminopyridin als Katalysatoren verwendet.

Das molare Verhältnis von Carbonaten der Formel III zu Oximen der Formel II kann zum Beispiel 0,5 bis 2,0, bevorzugt 0,8 bis 1,5 und besonders bevorzugt 0,8 bis 1 betragen. Katalytische Mengen einer Amidinbase oder Pyridinbase kann zum Beispiel 0,1 bis 10, bevorzugt 1 bis 10 und besonders bevorzugt 2 bis 6 Mol % bedeuten.

Das Verfahren kann in einem gegenüber den Reaktionspartnern inerten, organischen Lösungsmittel durchgeführt werden. Bevorzugt werden polare aprotische Lösungsmittel verwendet. Lösungsmittel werden mit Vorteil dann verwendet, wenn die Reaktionsprodukte isoliert und Lösungsmittel sowie Katalysator und überschüssige Ausgangsprodukte rezykliert und zurückgewonnen werden sollen. Durch Wahl der Lösungsmittel ( Siedepunkte ) können hierfür optimale Bedingungen eingestellt werden. Beispiele von organischen Lösungsmitteln sind aromatische oder aliphatische Lösungsmittel wie Benzol, Toluol, Xylol, Mesytilen, Hexan, Heptan Octan, Cyclohexan; aliphatische und aromatische Halogenkohlenwasserstoffe wie Methylenchlorid, Chloroform, Kohlenstofftetrachlorid, Trichlorethan, Chlorbenzol, Dichlorbenzol; Ether wie Diethylether, Dibutylether, Diisobutylether, Tetrahydrofuran und Dioxan; ferner Dimethylsulfoxid und Säureamidderivate, wie N, N-Dimethylformamid, N, N-Dimethylacetamid und N-Methyl-2-pyrrolidon; und Carbonsäureester wie Ethylacetat. Bevorzugt sind Toluol, Xylol, Dichlorbenzol, Chlorbenzol oder Dimethylformamid.

Die Konzentration an Oxim und Carbonat in der Reaktionsmischung kann bei Mitverwendung eines Lösungsmittels 20 bis 60, bevorzugt 25 bis 50 Vol%, bezogen auf das Lösungsmittel betragen.

Das Verfahren kann zum Beispiel bei einer Temperatur von 80° C bis 200° C, vorzugsweise von 90° C bis 150° C durchgeführt werden.
Das Verfahren kann ferner unter Normaldruck oder leichtem Über-oder Unterdruck durchgeführt werden.

Im einzelnen kann man zum Beispiel so vorgehen, dass man das Oxim oder die Lösung des Oxims langsam zu dem erwärmten oder siedenden Carbonat, bzw. Lösung des Carbonats zugibt. Dann lässt man noch eine gewisse Zeit nachreagieren, z. B. 0,5 bis 2 Stunden und isoliert das Reaktionsprodukt zum Beispiel mittels Rektifikation.

Eine andere Möglichkeit ist zum Beispiel, dass zu dem aus Hydroxylaminsulfat und Keton in situ hergestellten Oxim die Carbonatlösung zugegeben wird und nach einer Reaktionszeit von z. B. 0,5 bis 2 Stunden das Reaktionsprodukt isoliert wird.

Ein weiterer Vorteil des erfindungsgemässen Verfahrens ist, dass die entstehenden ω-Hydroxyalkyloxime nicht isoliert werden müssen, sondern als Lösung im organischen Lösungsmittel direkt für Folgereaktionen eingesetzt werden können. Dadurch wird die Herstellung von zum Beispiel herbizid wirksame Verbindungen wesentlich vereinfacht.

Die folgenden Beispiele erläutern die Erfindung.

### Beispiel 1.

In einem mit Rückflusskühler, Thermometer, Tropftrichter und Rührer versehenen Reaktionsgefäss, das aussenseitig mit einem Temperiermantel umgeben ist, wird eine Lösung von 160g (1,81 Mol) Ethylencarbonat und 11,02g (0,07 Mol) 1,8-Diazabicyclo[5.4.0]undec-7-en in 220g Toluol zum Rückfluss erhitzt. Zu dieser Lösung wird über einen Zeitraum von 2 Stunden eine Lösung von 146,2g (2,00 Mol) Acetonoxim in 217g Toluol zugetropft. Danach wird die Reaktionslösung noch 1 Stunde unter Rückfluss gehalten, bis die CO₂-Gasentwicklung beendet ist und eine vollständige Umsetzung von Ethylencarbonat erreicht ist. Man erhält 722g einer 24,4% igen toluolischen Oximglykol Lösung, was einer Ausbeute von 83,1% entspricht. Das reine Oximglykol wird durch anschliessende zweimalige Rektifikation je nach gewünschter Reinheit in einer Ausbeute von 65% bis 75% erhalten.

### Beispiel 2

Man verfährt wie in Beispiel 1 setzt jedoch 13,82g (0,09 Mol)1,8-Diazabicyclo[5.4.0]undec-7-en in 220g Toluol ein und erhitzt zum Rückfluss. Zu dieser Lösung wird über einen Zeitraum von 2 Stunden eine Lösung von 146,2g (2,00 Mol) Acetonoxim in 330g Toluol zugetropft. Man verfährt weiter wie in Beispiel 1 angegeben und erhält 813g einer 21,4 %igen toluolischen Oximgykol Lösung, was einer Ausbeute von 82,1% entspricht.Das reine Oximglykol wird durch anschliessende zweimalige Rektifikation je nach gewünschter Reinheit in einer Ausbeute von 65% bis 75% erhalten.

## Patentansprüche

1. Verfahren zur Hydroxyalkylierung von Aldoximen und Ketoximen durch Umsetzung dieser Oxime mit unsubstituiertem oder substituiertem Ethylen- oder Propylencarbonat in Gegenwart eines Katalysators, dadurch gekennzeichnet, dass man katalytische Mengen einer N-alkylierten, stabilen, organischen Amidinbase oder eines mit sekundärem Amin substituierten Pyridins verwendet.

2. Verfahren nach Anspruch 1, dadurch gekennzeichnet, dass die Amidinbase das Strukturelement -C-N=C-N-C- und insgesamt 4 bis 20 C-Atome enthält.

3. Verfahren zur Herstellung von Verbindungen der Formel I nach Anspruch 1, 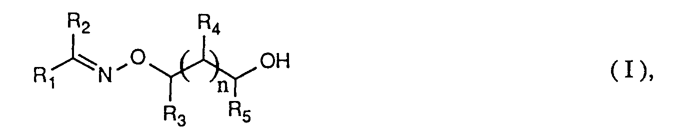 worin R₁ und R₂ unabhängig voneinander Wasserstoff, C₁-C₈-Alkyl, C₃-C₈-Cycloalkyl, C₁-C₈-Halogenalkyl, unsubstituiertes oder mit C₁-C₈ Alkyl oder Halogen substituiertes Phenyl, Benzyl, Phenylethyl bedeuten, oder R₁ und R₂ zusammen unsubstituiertes oder mit C₁-C₈-Alkyl oder Halogen substituiertes C₂-C₇-Alkylen bedeuten, R₃, R₄ und R₅ unabhängig voneinander Wasserstoff oder C₁-C₈ Alkyl sind und n eine Zahl 0 oder 1 ist, durch Umsetzung einer Verbindung der Formel II 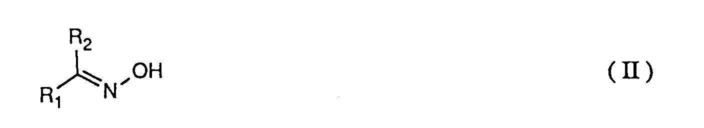 mit einer Verbindung der Formel III 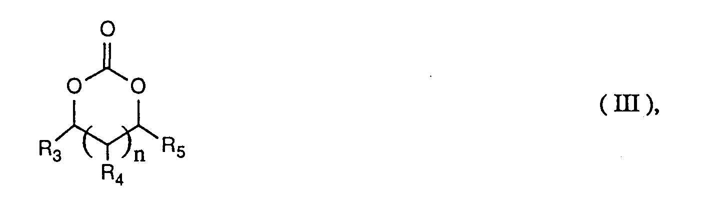 worin R₃, R₄, R₅ und n die vorstehend angegebene Bedeutung haben.

4. Verfahren nach Anspruch 3, dadurch gekennzeichnet, dass R₁ und R₂ unabhängig voneinander Wasserstoff oder C₁-C₈-Alkyl, oder zusammen unsubstituiertes C₂-C₇-Alkylen bilden, R₃ und R₅ Wasserstoff oder C₁-C₈ Alkyl bedeuten, wobei n für 0 steht.

5. Verfahren nach Anspruch 3, dadurch gekennzeichnet, dass, R₁ und R₂ Methyl, Ethyl oder Propyl sind, R₃ und R₄ Wasserstoff bedeuten und n für 0 steht.

6. Verfahren nach Anspruch 1, dadurch gekennzeichnet, dass man katalytische Mengen einer stabilen organischen Amidinbase der Formel IV oder eines mit sekundärem Amin substituierten Pyridins der Formel V 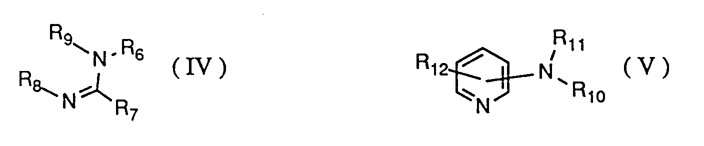 verwendet, worin R₆, R₇, R₈ und R₉ unabhängig voneinander C₁-C₈-Alkyl oder C₃-C₈-Cycloalkyl bedeuten, oder R₆ und R₇ zusammen einen Kohlenwasserstoffrest mit 3 bis 6 C-Atomen bilden und R₈ und R₉ die vorstehend angegebene Bedeutung haben,
oder R₈ und R₉ zusammen einen Kohlenwasserstoffrest mit 2 bis 6 C-Atomen bilden und R₆ und R₇ C₁-C₈-Alkyl oder C₃-C₈-Cycloalkyl bedeuten,
oder R₆ und R₇ einen Kohlenwasserstoffrest mit 3 bis 6 C-Atomen bilden sowie R₈ und R₉ zusammen einen Kohlenwasserstoffrest mit 2 bis 6 C-Atomen bilden,
R₁₀ und R₁₁ unabhängig voneinander C₁-C₁₂-Alkyl sind und R₁₂ für Wasserstoff, C₁-C₈-Alkyl, C₃-C₈-Cycloalkyl oder C₁-C₈-Halogenalkyl steht.

7. Verfahren nach Anspruch 6, dadurch gekennzeichnet, dass als Katalysator eine Verbindung der Formel IV oder V verwendet wird, wobei R₆ und R₇ zusammen einen Kohlenwasserstoffrest mit 3 bis 6 C-Atomen bilden und R₈ und R₉ C₁-C₈-Alkyl oder C₅-C₆-Cycloalkyl sind, oder R₈ und R₉ zusammen einen Kohlenwasserstoffrest mit 2 bis 6 C-Atomen bilden und R₆ und R₇ C₁-C₈-Alkyl oder C₅-C₆-Cycloalkyl bedeuten, oder R₆ und R₇ einen Kohlenwasserstoffrest mit 3 bis 6 C-Atomen bilden sowie R₈ und R₉ zusammen einen Kohlenwasserstoffrest mit 2 bis 6 C-Atomen bilden,
R₁₀ und R₁₁ unabhängig voneinander C₁-C₄-Alkyl bedeuten und R₁₂ für Wasserstoff, C₁-C₄-Alkyl, C₅-C₆-Cycloalkyl oder C₁-C₄-Halogenalkyl steht.

8. Verfahren nach Anspruch 6, dadurch gekennzeichnet, dass als Katalysator eine Amidinbase der Formel IV verwendet wird, wobei R₆ und R₇ zusammen einen gesättigten Kohlenwasserstoffrest mit 3 bis 6 C-Atomen bilden und R₈ und R₉ zusammen einen gesättigten Kohlenwasserstoffrest mit 2 bis 6 C-Atomen bilden.

9. Verfahren nach Anspruch 6, dadurch gekennzeichnet, dass als Katalysator eine Verbindungen der Formel V verwendet wird, wobei R₁₂ Wasserstoff oder C₁-C₈-Alkyl bedeutet, und R₁₀ und R₁₁ gleich sind und für C₁-C₄-Alkyl stehen.

10. Verfahren nach Anspruch 6, dadurch gekennzeichnet, dass als Katalysator eine Verbindungen der Formel V verwendet wird, wobei R₁₂ Wasserstoff ist und die Gruppe -NR₁₀R₁₁ in 2 oder 4 Stellung gebunden ist, wobei R₁₀ und R₁₁ C₁-C₄-Alkyl sind.

11. Verfahren nach Anspruch 6, dadurch gekennzeichnet, dass als Katalysator 1,5-Diazabicyclo[4.3.0]non-5-en oder 1,8-Diazabicyclo[5.4.0]undec-7-en oder 2-Dimethylaminopyridin oder 4-Dimethylaminopyridin verwendet wird.

12. Verfahren nach Anspruch 1, dadurch gekennzeichnet, dass das molare Verhältnis von Carbonaten der Formel III zu Oximen der Formel II 0,5 bis 2,0 beträgt.

13. Verfahren nach Anspruch 12, dadurch gekennzeichnet, dass das molare Verhältnis von Carbonaten der Formel III zu Oximen der Formel II 0,8 bis 1,5 beträgt.

14. Verfahren nach Anspruch 12, dadurch gekennzeichnet, dass das molare Verhältnis von Carbonaten der Formel III zu Oximen der Formel II 0,8 bis 1,0 beträgt.

15. Verfahren nach Anspruch 1, dadurch gekennzeichnet, dass die katalytischen Mengen von Verbindungen der Formel IV oder V 0,1 bis 10 Mol %, bezogen auf das eingesetzte Oxim betragen.

16. Verfahren nach Anspruch 6, dadurch gekennzeichnet, dass die katalytischen Mengen von Verbindungen der Formel IV oder V 1 bis 10 Mol %, bezogen auf das eingesetzte Oxim betragen.

17. Verfahren nach Anspruch 6, dadurch gekennzeichnet, dass die katalytischen Mengen von Verbindungen der Formel IV oder V 2 bis 6 Mol %, bezogen auf das eingesetzte Oxim betragen.

18. Verfahren nach Anspruch 6, dadurch gekennzeichnet, dass es in einem gegenüber den Reaktionspartnern inerten, organischen Lösungsmittel durchgeführt wird.

19. Verfahren nach Anspruch 18, dadurch gekennzeichnet, dass die Konzentration an Oxim und Carbonat bei Mitverwendung eines Lösungsmittels 20 bis 60 Vol%, bezogen auf das Lösungsmittel beträgt.

20. Verfahren nach Anspruch 1, dadurch gekennzeichnet, dass es bei einer Temperatur von 80° C bis 200° C durchgeführt wird.

21. Verfahren nach Anspruch 20, dadurch gekennzeichnet, dass es bei einer Temperatur von 90° C bis 150° C durchgeführt wird.

## Claims

1. A process for the hydroxyalkylation of aldoximes and ketoximes by reacting said oximes with unsubstituted or substituted ethylene carbonate or propylene carbonate in the presence of a catalyst, which comprises the use of catalytic amounts of an N-alkylated, stable, organic amidine base or of a pyridine which is substituted with secondary amine.

2. A process according to claim 1, wherein the amidine base contains the structural element -C-N=C-N-C- and contains a total of 4 to 20 carbon atoms.

3. A process according to claim 1 for the preparation of a compound of formula I 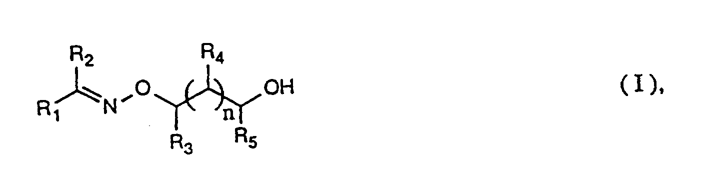 wherein R₁ and R₂ are each independently of the other hydrogen, C₁-C₈alkyl, C₃-C₈cycloalkyl, C₁-C₈haloalkyl, unsubstituted or C₁-C₈alkyl- or halogen-substituted phenyl, benzyl or phenylethyl, or R₁ and R₂, taken together, are unsubstituted or C₁-C₈alkyl- or halogen-substituted C₂-C₇alkylene, R₃, R₄ and R₅ are each independently of one another hydrogen or C₁-C₈alkyl, and
n is 0 or 1, by reacting a compound of formula II 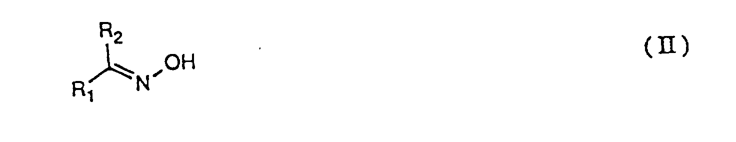 with a compound of formula III 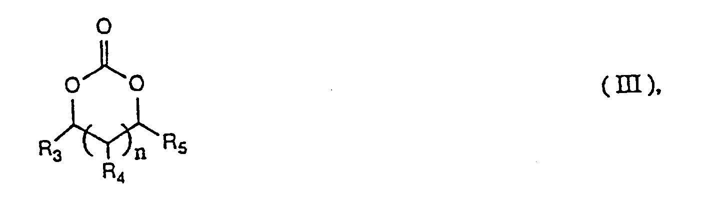 wherein R₃, R₄, R₅ and n are as defined above.

4. A process according to claim 3, wherein R₁ and R₂ are each independently of the other hydrogen or C₁-C₈alkyl, or taken together form unsubstituted C₂-C₇alkylene, R₃ and R₅ are hydrogen or C₁-C₈alkyl, and n is 0.

5. A process according to claim 3, wherein R₁ and R₂ are methyl, ethyl or propyl, R₃ and R₅ are hydrogen, and n is 0.

6. A process according to claim 1, which comprises the use of catalytic amounts of a stable organic amidine base of formula IV or of a pyridine which is substituted with secondary amine, of formula V 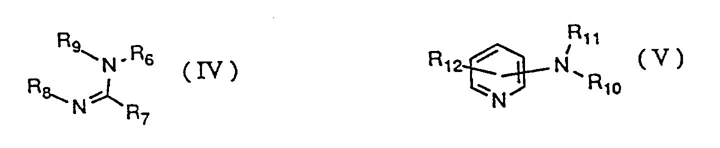 wherein R₆, R₇, R₈ and R₉ are each independently of one another C₁-C₈alkyl or C₃-C₈cycloalkyl, or R₆ and R₇ taken together form a hydrocarbon radical containing 3 to 6 carbon atoms, and R₈ and R₉ are as defined above, or R₈ and R₉, taken together, form a hydrocarbon radical containing 2 to 6 carbon atoms, and R₆ and R₇ are C₁-C₈alkyl or C₃-C₈cycloalkyl, or R₆ and R₇, taken together, form a hydrocarbon radical containing 3 to 6 carbon atoms, and R₈ and R₉, taken together, form a hydrocarbon radical containing 2 to 6 carbon atoms,
R₁₀ and R₁₁ are each independently of the other C₁-C₁₂alkyl, and R₁₂ is hydrogen, C₁-C₈alkyl, C₃-C₈cycloalkyl or C₁-C₈haloalkyl.

7. A process according to claim 6, wherein the catalyst used is a compound of formula IV or V, where R₆ and R₇ taken together form a hydrocarbon radical containing 3 to 6 carbon atoms,
and R₈ and R₉ are C₁-C₈alkyl or C₅-C₆cycloalkyl, or R₈ and R₉, taken together, form a hydrocarbon radical containing 2 to 6 carbon atoms, and
R₆ and R₇ are C₁-C₈alkyl or C₅-C₆cycloalkyl, or R₆ and R₇ form a hydrocarbon radical containing 3 to 6 carbon atoms, and R₈ and R₉, taken together, form a hydrocarbon radical containing 2 to 6 carbon atoms,
R₁₀ and R₁₁ are each independently of the other C₁-C₄alkyl and R₁₂ is hydrogen, C₁-C₄alkyl, C₅-C₆cycloalkyl or C₁-C₄haloalkyl.

8. A process according to claim 6, wherein the catalyst used is an amidine base of formula IV, where R₆ and R₇ together form a saturated hydrocarbon radical of 3 to 6 carbon atoms, and R₈ and R₉ together form a saturated hydrocarbon radical of 2 to 6 carbon atoms.

9. A process according to claim 6, wherein the catalyst used is a compound of formula V, where R₁₂ is hydrogen or C₁-C₈alkyl, and R₁₀ and R₁₁ are identical and are C₁-C₄alkyl.

10. A process according to claim 6, wherein the catalyst used is a compound of formula V, where R₁₂ is hydrogen and the group -NR₁₀R₁₁ is in position 2 or 4, and R₁₀ and R₁₁ are C₁-C₄alkyl.

11. A process according to claim 6, wherein the catalyst used is 1,5-diazabicyclo[4.3.0]non-5-ene or 1,8-diazabicyclo[5.4.0]undec-7-ene or 2-dimethylaminopyridine and 4-dimethylaminopyridine.

12. A process according to claim 1, wherein the molar ratio of carbonate of formula III to oxime of formula II is from 0.5 to 2.0.

13. A process according to claim 12, wherein the molar ratio of carbonate of formula III to oxime of formula II is from 0.8 to 1.5.

14. A process according to claim 12, wherein the molar ratio of carbonate of formula III to oxime of formula II is from 0.8 to 1.0.

15. A process according to claim 1, wherein the catalytic amount of the compound of formula IV or V is from 0.1 to 10 mol %, based on the oxime employed.

16. A process according to claim 6, wherein the catalytic amount of the compound of formula IV or V is from 1 to 10 mol %, based on the oxime employed.

17. A process according to claim 6, wherein the catalytic amount of the compound of formula IV or V is from 2 to 6 mol %, based on the oxime employed.

18. A process according to claim 6, which is carried out in an organic solvent which is inert to the reactants.

19. A process according to claim 18, wherein the concentration of oxime and carbonate, when concurrently using a solvent, is from 20 to 60% by volume, based on the solvent.

20. A process according to claim 1, which is carried out at a temperature from 80 to 200°C.

21. A process according to claim 20, which is carried out at a temperature from 90 to 150°C.

## Revendications

1. Procédé permettant l'hydroxyalcoylation des aldoximes et cétoximes par réaction de ces oximes avec le carbonate d'éthylène ou de propylène non substitué ou substitué, en présence d'un catalyseur, caractérisé en ce que l'on utilise des quantités catalytiques d'une base amidine organique, stable, N-alkylée ou d'une pyridine substituée par une amine secondaire.

2. Procédé selon la revendication 1, caractérisé en ce que la base amidine contient l'élément structural -C-N=C-N-C- et, au total, 4 à 20 atomes de C.

3. Procédé pour la préparation des composés de formule I selon la revendication 1 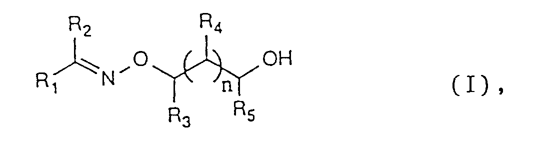 où R₁ et R₂ représentent, indépendamment l'un de l'autre, l'hydrogène, un alkyle en C₁-C₈, un cycloalkyle en C₃-C₈, un halogénoalkyle en C₁-C₈, un phényle, un benzyle, un phényléthyle non substitué ou substitué par un alkyle en C₁-C₈ ou par un halogène, ou R₁ et R₂ représentent ensemble un alkylène en C₂-C₇ non substitué ou substitué par un alkyle en C₁-C₈ ou par un halogène, R₃, R₄ et R₅ représentent, indépendamment les uns des autres, l'hydrogène ou un alkyle en C₁-C₈ et n est égal à 0 ou 1, par réaction d'un composé de formule II 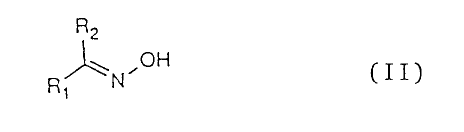 avec un composé de formule III 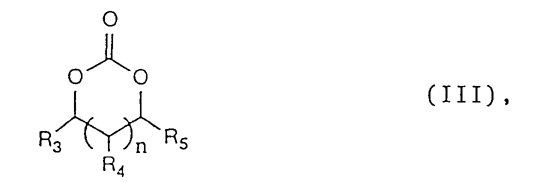 où R₃, R₄, R₅ et n ont les significations précédemment indiquées.

4. Procédé selon la revendication 3, caractérisé en ce que R₁ et R₂ représentent, indépendamment l'un de l'autre, l'hydrogène ou un alkyle en C₁-C₈ ou forment ensemble un alkylène en C₂-C₇ non substitué, R₃ et R₅ représentent l'hydrogène ou un alkyle en C₁-C₈, n étant égal à 0.

5. Procédé selon la revendication 3, caractérisé en ce que R₁ et R₂ sont le méthyle, l'éthyle ou le propyle, R₃ et R₄ représentent l'hydrogène et n est égal à 0.

6. Procédé selon la revendication 1, caractérisé en ce que l'on utilise des quantités catalytiques d'une base amidine organique, stable, de formule IV ou d'une pyridine substituée par une amine secondaire de formule V 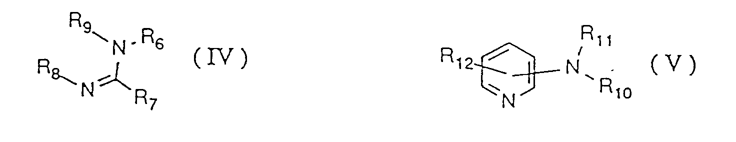 où R₆, R₇, R₈ et R₉ représentent, indépendamment les uns des autres, un alkyle en C₁-C₈ ou un cycloalkyle en C₃-C₈, ou R₆ et R₇ forment ensemble un reste hydrocarboné comportant 3 à 6 atomes de C et R₈ et R₉ ont les significations précédemment indiquées, ou R₈ et R₉ forment ensemble un reste hydrocarboné comportant 2 à 6 atomes de C et R₆ et R₇ représentent un alkyle en C₁-C₈ ou un cycloalkyle en C₃-C₈, ou R₆ et R₇ forment ensemble un reste hydrocarboné comportant 3 à 6 atomes de C, et R₈ et R₉ forment ensemble un reste hydrocarboné comportant 2 à 6 atomes de C, R₁₀ et R₁₁ représentent, indépendamment l'un de l'autre, un alkyle en C₁-C₁₂ et R₁₂ représente l'hydrogène, un alkyle en C₁-C₈, un cycloalkyle en C₃-C₈ ou un halogénoalkyle en C₁-C₈.

7. Procédé selon la revendication 6, caractérisé en ce que l'on utilise, comme catalyseur, un composé de formule IV ou V où R₆ et R₇ forment ensemble un reste hydrocarboné comportant 3 à 6 atomes de C et R₈ et R₉ sont un alkyle en C₁-C₈ ou un cycloalkyle en C₅-C₆ ou R₈ et R₉ forment ensemble un reste hydrocarboné comportant 2 à 6 atomes de C et R₆ et R₇ représentent un alkyle en C₁-C₈ ou un cycloalkyle en C₅-C₆, ou R₆ et R₇ forment ensemble un reste hydrocarboné comportant 3 à 6 atomes de C, et R₈ et R₉ forment ensemble un reste hydrocarboné comportant 2 à 6 atomes de C, R₁₀ et R₁₁ représentent, indépendamment l'un de l'autre, un alkyle en C₁-C₄ et R₁₂ représente l'hydrogène, un alkyle en C₁-C₄, un cycloalkyle en C₅-C₆ ou un halogénoalkyle en C₁-C₄.

8. Procédé selon la revendication 6, caractérisé en ce que l'on utilise, à titre de catalyseur, une base amidine de formule IV où R₆ et R₇ forment ensemble un reste hydrocarboné saturé comportant 3 à 6 atomes de C et R₈ et R₉ forment ensemble un reste hydrocarboné saturé comportant 2 à 6 atomes de C.

9. Procédé selon la revendication 6, caractérisé en ce que l'on utilise, à titre de catalyseur, un composé de formule V où R₁₂ représente l'hydrogène ou un alkyle en C₁-C₈ et R₁₀ et R₁₁ sont identiques et représentent un alkyle en C₁-C₄.

10. Procédé selon la revendication 6, caractérisé en ce que l'on utilise, à titre de catalyseur, un composé de formule V où R₁₂ est l'hydrogène et le groupe -NR₁₀R₁₁ est lié en position 2 ou 4, R₁₀ et R₁₁ étant un alkyle en C₁-C₄.

11. Procédé selon la revendication 6, caractérisé en ce que l'on utilise, à titre de catalyseur, le 1,5-diazabicyclo[4.3.0]non-5-ène ou le 1,8-diazabicyclo[5.4.0]undéc-7-ène ou la 2-diméthylaminopyridine ou la 4-diméthylaminopyridine.

12. Procédé selon la revendication 1, caractérisé en ce que le rapport molaire des carbonates de formule III aux oximes de formule II est compris entre 0,5 et 2,0.

13. Procédé selon la revendication 12, caractérisé en ce que le rapport molaire des carbonates de formule III aux oximes de formule II est compris entre 0,8 et 1,5.

14. Procédé selon la revendication 12, caractérisé en ce que le rapport molaire des carbonates de formule III aux oximes de formule II est compris entre 0,8 et 1,0.

15. Procédé selon la revendication 1, caractérisé en ce que les quantités catalytiques des composés de formule IV ou V sont comprises entre 0,1 et 10% en mole, rapporté à l'oxime utilisée.

16. Procédé selon la revendication 6, caractérisé en ce que les quantités catalytiques des composés de formule IV ou V sont comprises entre 1 et 10% en mole, rapporté à l'oxime utilisée.

17. Procédé selon la revendication 6, caractérisé en ce que les quantités catalytiques des composés de formule IV ou V sont comprises entre 2 et 6% en mole, rapporté à l'oxime utilisée.

18. Procédé selon la revendication 6, caractérisé en ce qu'il est exécuté dans un solvant organique, inerte vis-à-vis des partenaires réactionnels.

19. Procédé selon la revendication 18, caractérisé en ce que la concentration en oxime et en carbonate, dans le cas où l'on utilise un solvant, est comprise entre 20 et 60% en volume, rapporté au solvant.

20. Procédé selon la revendication 1, caractérisé en ce qu'il est exécuté à une température allant de 80°C à 200°C.

21. procédé selon la revendication 20, caractérisé en ce qu'il est exécuté à une température allant de 90°C à 150°C.
